# EUROPEAN PATENT APPLICATION

(11) **EP 4 458 950 A1**
(43) Date of publication of application: **06.11.2024**
(21) Application number: 22915080.0
(22) Date of filing: 29.12.2022
(51) Int. Cl.: C12N 1/20, C12N 1/02, C12R 1/19, C12R 1/07, C12R 1/225

(54) **METHOD FOR COLLECTING MICROBIAL BODIES FROM FERMENTATION SOLUTION SUITABLE FOR AUTOMATIC OPERATION**

(30) Priority: 31.12.2021 CN 202111660921
(71) Applicant: WuXi Biologics Ireland Limited, Dundalk, Co Louth A91 X56F (IE)
(72) Inventor: ZHI, Yan, Wuxi, Jiangsu 214092 (CN); CHEN, Xiaoyue, Wuxi, Jiangsu 214092 (CN); ZHOU, Weichang, Wuxi, Jiangsu 214092 (CN); CHEN, Zhisheng, Wuxi, Jiangsu 214092 (CN)
(74) Representative: Modiano, Gabriella Diana
(86) International application number: PCT/CN2022/143490
(87) International publication number: WO 2023/125831

(57) **Abstract**

Disclosed is a method for collecting bacteria from bacterial or fungal fermentation culture broth, the method comprising the steps of: a) mixing bacterial or fungal fermentation culture medium with a carboxylated chitosan to obtain a mixture; optionally, b) adding a sodium alginate solution to the mixture and mixing homogeneously; and c) precipitating to obtain bacterial or fungal cell precipitate or culture supernatant.

## Description

### (1) TECHNICAL FIELD

The present invention belongs to the technical field of biochemical engineering post-processing, and in particular, relates to a method for recovering bacteria from fermentation broth of bacteria and fungi suitable for automated operation.

### (2) BACKGROUND

In 2021, 24 of the top 30 best-selling drugs in the world are biological drugs. The speed and success of biological drug development are closely related to the ability to quickly obtain corresponding protein samples for the studies of protein structure and *in vitro* and *in vivo* activities. At present, the transient expression process of mammalian cells is the major way to obtain study-use proteins. The amount of plasmid DNA required for this process is generally in the order of milligram to gram, and depending on the size of the preliminary candidate molecule library, the number of protein samples that need to be produced generally ranges from dozens to thousands, and correspondingly, the number of plasmid DNA required is also be in the order of dozens to thousands. In the process of accelerating the development of biological drugs, how to quickly obtain a large amount of plasmid DNA required for protein production is an problem that urgently needs to be solved.

Taking bacteria as an example, *Escherichia coli* (*E. coli*) is an important genetic engineering host in modern biotechnology. It has the advantages of clear genetic background, fast growth rate, simple genetic operation, and easiness of production process amplification. It is widely used in the production of plasmid DNA, recombinant proteins, enzymes, and other biological products at the laboratory level or industrial scale. When *Escherichia coli* is used as a host cell to produce plasmid DNA, it is necessary to recover *Escherichia coli* cells from the culture broth and then extract the required plasmid DNA from the *Escherichia coli* cells. There are two conventional methods for recovering *Escherichia coli* cells from fermentation broth: high-speed centrifugation and membrane filtration. The high-speed centrifugation is usually used for the preparation of microgram to milligram levels of plasmid DNA in the laboratory, wherein the number of samples processed in a batch is limited, more manual operations are required, and the throughput and scale are difficult to scale up. The membrane filtration method can process samples on a large scale in a single process, which is suitable for large-scale processing of same type of samples, but there are risks of membrane clogging, damage, contamination, *etc.*; the cost of consumables is also high, and it is difficult to achieve multi-sample, high-throughput processing. In addition, both the centrifugation and the membrane filtration rely heavily on manual operations, and as the number of samples to be processed in the batch(es) increases, there is a corresponding need to multiply the equipment(s) and personnel investment. Therefore, there is an urgent need to establish a method for collecting *Escherichia coli* cells that is suitable for high-throughput and automated processing.

Chemical flocculation can be used to separate bacteria from the culture broth and is a low-cost alternative to centrifugation and membrane filtration. A published Chinese patent (CN109439584A) shows that chitosan and sodium alginate can concentrate *E*. *coli* fermentation broth through flocculation to enrich bacteria. However, this method only studies the recovery of *E. coli* cells and does not study the effect of sedimentation on cell integrity, product, and more importantly, subsequent plasmid recovery.

Chitosan is a linear amino polysaccharide prepared by deacetylation of chitin in hot alkali solution. Its chemical name is β-(1, 4)-2-amino-2-deoxy-D-glucan, with the molecular formula of (C6H11NO4)n, and its structural formula is as follows:

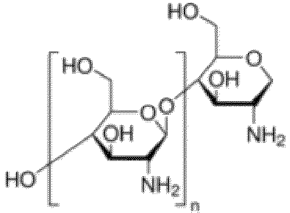

Chitosan is insoluble in water and organic solvents, but soluble in diluted acid solutions. The insolubility of chitosan in water makes the process of its use complicated, and therefore, its application in flocculation for bacteria collection has not yet been widely realized.

Therefore, there is still a need in the art to develop a method for collecting bacteria cells that is simple to operate, has no effect on the subsequent plasmid production yield, does not rely on centrifuges or membrane filtration, and can be automated. This is of great significance for the high-throughput automated production of plasmid DNA and the rapid production of proteins required for the late-stage biopharmaceutical research and development.

### (3) SUMMARY OF THE INVENTION

Therefore, the object of the present invention is to provide an improved method for recovering bacteria from bacterial and fungal cell fermentation cultures while maintaining the integrity of their plasmid DNA and RNA and efficiently extracting protein and nucleic acid products.

Therefore, the present invention provides a method for collecting bacteria from bacterial or fungal fermentation culture broth, characterized in that the method comprises the steps of:
a) mixing the fermentation culture broth obtained by bacterial or fungal fermentation cultures with the carboxylated chitosan to obtain a mixture;
b) adding sodium alginate solution to the mixture and mixing homogeneously; and
c) precipitating to obtain a bacterial precipitate.

In another aspect of the present invention, the step b) in the above method is omitted, and thus a method for collecting bacterial precipitate or fermentation broth supernatant from bacterial or fungal fermentation broth is provided in this aspect, the method comprising the steps of:
a) mixing a fermentation broth obtained by bacterial or fungal fermentation culture with the carboxylated chitosan to obtain a mixture; and c) Precipitating to obtain a bacterial precipitate or fermentation broth supernatant.

A preferred embodiment of this aspect also includes the step d) of recovering the bacteria after the step c).

In a specific embodiment, the bacteria are selected from *Bacillus, Escherichia coli, Lactobacillus, Lactococcus,* preferably *Escherichia coli* or *Bacillus subtilis*; the fungi are selected from yeast, preferably baker's yeast, feed yeast, *Saccharomyces cerevisiae, Pichia pastoris*; *Candida*; mucedine, more preferably *Rhizopus, Aspergillus,* most preferably *Saccharomyces cerevisiae* and *Pichia pastoris.*

In a most preferred embodiment, the bacterium is *Escherichia coli,* and/or the fungus is *Pichia pastoris.*

In one embodiment, the amount of carboxylated chitosan mixed in step a) is 1%-32% by weight, preferably 1-16% by weight, more preferably 4%-8% by weight, and most preferably 8% by weight of the fermentation broth.

In a specific embodiment, the amount of sodium alginate added in step b) is 1%- 16% by weight, preferably 1%-8% by weight, more preferably 4%-8% by weight, and most preferably 8% by weight of the fermentation broth.

In another specific embodiment, the amount of carboxylated chitosan and sodium alginate added to the fermentation broth is 8% by weight of the fermentation broth respectively; or the amount of carboxylated chitosan added to the fermentation broth is 8% by weight of the fermentation medium, and without sodium alginate. In a more preferred embodiment, the fermentation broth is preferably selected from *Escherichia coli* fermentation broth and *Pichia pastoris* fermentation broth.

In another preferred embodiment, the weight ratio of the added carboxylated chitosan to sodium alginate is 1:4-4:1, preferably 1:2-2:1, and more preferably 1:1.

In a specific embodiment, the carboxylated chitosan is selected from chitosan in which the amino group (-NH₂ ), or the hydroxyl group (-OH), or the amino group (-NH₂) and the hydroxyl group (-OH) are modified by a carboxylation group, wherein the carboxylation group is a C1-C6 linear or branched alkyl group substituted with 1-3 carboxyl groups, preferably with 3 carboxyl groups, more preferably 2 carboxyl groups, and most preferably 1 carboxyl group; the linear or branched alkyl group preferably has 1-6 carbon atoms.

In another specific embodiment, the *E*. *coli* strains comprise *E*. *coli* TOPIC, *E*. *coli* JM109, *E*. *coli* HB101, *E*. *coli* DH5 α, *E. coli* BL21 (DE3), and other derivatives of *E. coli* K strains and B strains. In a preferred embodiment, the yeast strains comprise *Saccharomyces cerevisiae, Pichia pastoris.*

In another specific embodiment, the recovery in step d) is selected from pouring the culture medium or pouring the culture medium by an automated robotic arm to obtain the bacteria, using physical or chemical method(s) to break the bacteria to release the DNA or RNA in the bacteria, adsorbing the DNA or RNA by magnetic beads, and washing and purifying the DNA or RNA with a washing solution, preferably pouring, more preferably manually pouring the culture medium or pouring the culture medium by an automated robotic arm.

In a specific embodiment of this aspect, after obtaining the bacteria, the protein can also be extracted from the bacteria.

In another preferred embodiment, the recovery of culture supernatant in step d) is by pouring the culture medium or pouring the culture medium by an automated robotic arm to obtain the culture supernatant, and then purifying the DNA or protein or small molecule metabolites in the supernatant.

In still another aspect, the *E. coli* fermentation medium is selected from LB broth medium, and the yeast fermentation medium is selected from YPD medium.

Therefore, the method for recovering bacteria from bacterial and fungal fermentation cultures of the present invention offers following advantages:
1. The use of water-soluble carboxylated chitosan and sodium alginate effectively facilitates the bacterial sedimentation in the fermentation broth, which is straightforward, cost-effective, and eliminates the need for centrifugation or membrane filtration.
2. After the fermentation broth is treated with carboxylated chitosan and sodium alginate, the fermentation supernatant can be removed by pouring to obtain the sedimented bacteria. This process can be automated by a robotic arm.
3. The bacteria precipitate obtained by sedimentation is relatively dispersed and does not form clump or flocs, which is beneficial for the lysis of bacteria and subsequent extraction of intracellular plasmid DNA, RNA and protein.
4. After the fermentation broth is treated with carboxylated chitosan and sodium alginate, the fermentation supernatant can be directly separated by the pouring method, and the supernatant can be used for subsequent biochemical separation process to obtain the target product (including but not limited to protein, alcohol, acid, ester, ketone and other biological metabolites).

Other features and advantages of various embodiments will be partially described in the following description, and in part will be apparent from the description, or can be understood through the practice of various embodiments. The objectives and other advantages of various embodiments will be realized and achieved through the elements and combinations particularly indicated in the description and the appended claims.

Unless otherwise indicated, the reagents, cells, and instruments/devices used in the present invention are commonly commercially available and publicly available.

### (4) DESCRIPTION OF THE DRAWINGS

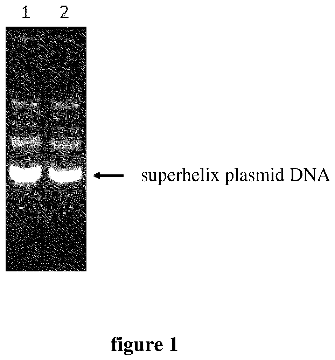

FIG. 1 shows the agarose gel electrophoretogram of plasmid DNA extracted from bacteria collected by centrifugation (lane 1) and plasmid DNA extracted from bacteria collected by carboxylated chitosan (lane 2).

### (5) DETAILED EMBODIMENTS

The following detailed description refers to certain embodiments of the present disclosure, which are further elaborated in the Examples section below. But it should be understood that they are not intended to limit the present invention to those embodiments. On the contrary, the present disclosure is intended to cover all alternatives, modifications, and equivalents of the present invention that can be encompassed by the appended claims.

The carboxylated chitosan used in the specific embodiments herein is selected from chitosan in which amino (-NH2), or hydroxyl (-OH), or amino (-NH₂) and hydroxyl (-OH) are modified by carboxylation groups, wherein the carboxylation groups are C1-C6 linear or branched alkyl groups substituted with 1-3 carboxyl groups, preferably 3 carboxyl groups, more preferably 2 carboxyl groups, and most preferably 1 carboxyl group; the linear or branched alkyl groups preferably have 1-6 carbon atoms. Carboxylated chitosan has good water solubility. Commercially available Carboxylated chitosan includes, for example, carboxylated chitosan produced by Aladdin Scientific, which can be prepared according to the methods disclosed in literatures such as "Synthesis of carboxylated chitosan and its adsorption properties for cadmium (II), lead (II) and copper (II) from aqueous solutions" ( KL Lv, YL Du, CM Wang;. Water Sci Technol 1 July 2009; 60 (2): 467-474. doi: https://doi.org/10.2166/wst.2009.369).

The fermentation cell culture of bacteria and fungi as described herein includes the technology in the art of using fermentation medium as a medium to culture bacteria and fungal cells under appropriate conditions to proliferate and produce the desired target product. The fermentation culture used herein can be laboratory-scale or industrial-production-scale.

The term "bacteria/bacterium" as used herein includes, but is not limited to, Gram-positive bacteria and Gram-negative bacteria, in particular bacteria commonly used in fermentation culture, such as *Bacillus, Escherichia coli, Lactobacillus, Lactococcus, etc.,* preferably *Escherichia coli* and *Bacillus subtilis.* The term "fungus" as used herein includes, but is not limited to, eukaryotic spore-forming microorganisms, including, but not limited to, yeast, preferably baker's yeast, feed yeast, *Saccharomyces cerevisiae* (brewer's yeast), *Pichia pastoris, etc.*; *Candida*; mucedine, such as *Rhizopus, Aspergillus, etc.,* preferably *Saccharomyces cerevisiae* and *Pichia pastoris.*

### Example 1: Extraction and analysis of E. coli cells

### 1. Reagents

LB broth medium (Sangong Biotech, A507002 ): 10 g/L tryptone, 10 g /L NaCl, 5 g /L yeast extract; chitosan solution: 1.5 g chitosan (Aladdin Scientific, C 105799) was dissolved in 100 mL of 1 % acetic acid solution (FISHER, A35-500 ) to obtain a 1.5% chitosan acetic acid solution.

Carboxylated chitosan solution: 1.5 g carboxylated chitosan (Aladdin Scientific, C 105800) was dissolved in 100 mL ultrapure water to obtain a 1.5% carboxylated chitosan aqueous solution.

Sodium alginate solution: 1.0 g of sodium alginate (Aladdin Scientific, S100127) was dissolved in 100 mL of ultrapure water to obtain a 1% sodium alginate aqueous solution.

Bacteria resuspension solution: 50 mM glucose (Shanghai Laboratory, 10010518), 25 mM Tris-HCl (pH8.0) (Shanghai Laboratory, 30188336), 10 mM EDTA (pH8.0) (Shanghai Laboratory, 10009717).

Lysis buffer: 0.2 mol/L NaOH (Shanghai Laboratory, 10019718), 1% (m/V) SDS (Shanghai Laboratory, 30166428).

Neutralization solution (100 mL): 60 mL 5M potassium acetate (Shanghai Test, 30154518), 11.5 mL glacial acetic acid (FISHER, A35-500 ), 28.5 mL distilled water.
RNase A: 10 mg/mL (Yeasen Biotechnology, 10405ES03).
pUC19 plasmid: Thermo Scientific SD0061
*E. coli* Top 10 strains: TIANGEN CB104

The sequence of the pUC19 plasmid is as follows:

### 1. pUC19 DNA sequence

> pUC19 (SEQ ID NO:1)

GAGATACCTACAGCGTGAGCTATGAGAAAGCGCCACGCTTCCCGAAGGGAGAAAGGC
GGACAGGTATCCGGTAAGCGGCAGGGTCGGAACAGGAGAGCGCACGAGGGAGCTTCCAGG
GGGAAACGCCTGGTATCTTTATAGTCCTGTCGGGTTTCGCCACCTCTGACTTGAGCGTCGAT
TTTTGTGATGCTCGTCAGGGGGGCGGAGCCTATGGAAAAACGCCAGCAACGCGGCCTTTTT
ACGGTTCCTGGCCTTTTGCTGGCCTTTTGCTCACATGTTCTTTCCTGCGTTATCCCCTGATTC
TGTGGATAACCGTATTACCGCCTTTGAGTGAGCTGATACCGCTCGCCGCAGCCGAACGACC
GAGCGCAGCGAGTCAGTGAGCGAGGAAGCGGAAGAGCGCCCAATACGCAAACCGCCTCTC
CCCGCGCGTTGGCCGATTCATTAATGCAGCTGGCACGACAGGTTTCCCGACTGGAAAGCGG
GCAGTGAGCGCAACGCAATTAATGTGAGTTAGCTCACTCATTAGGCACCCCAGGCTTTACA
CTTTATGCTTCCGGCTCGTATGTTGTGTGGAATTGTGAGCGGATAACAATTTCACACAGGAA
ACAGCTATGACCATGATTACGCCAAGCTTGCATGCCTGCAGGTCGACTCTAGAGGATCCCC
GGGTACCGAGCTCGAATTCACTGGCCGTCGTTTTACAACGTCGTGACTGGGAAAACCCTGG
CGTTACCCAACTTAATCGCCTTGCAGCACATCCCCCTTTCGCCAGCTGGCGTAATAGCGAAG
AGGCCCGCACCGATCGCCCTTCCCAACAGTTGCGCAGCCTGAATGGCGAATGGCGCCTGAT
GCGGTATTTTCTCCTTACGCATCTGTGCGGTATTTCACACCGCATATGGTGCACTCTCAGTA
CAATCTGCTCTGATGCCGCATAGTTAAGCCAGCCCCGACACCCGCCAACACCCGCTGACGC
GCCCTGACGGGCTTGTCTGCTCCCGGCATCCGCTTACAGACAAGCTGTGACCGTCTCCGGG
AGCTGCATGTGTCAGAGGTTTTCACCGTCATCACCGAAACGCGCGAGACGAAAGGGCCTCG
TGATACGCCTATTTTTATAGGTTAATGTCATGATAATAATGGTTTCTTAGACGTCAGGTGGC
ACTTTTCGGGGAAATGTGCGCGGAACCCCTATTTGTTTATTTTTCTAAATACATTCAAATAT
GTATCCGCTCATGAGACAATAACCCTGATAAATGCTTCAATAATATTGAAAAAGGAAGAGT
ATGAGTATTCAACATTTCCGTGTCGCCCTTATTCCCTTTTTTGCGGCATTTTGCCTTCCTGTT
TTTGCTCACCCAGAAACGCTGGTGAAAGTAAAAGATGCTGAAGATCAGTTGGGTGCACGAG
TGGGTTACATCGAACTGGATCTCAACAGCGGTAAGATCCTTGAGAGTTTTCGCCCCGAAGA
ACGTTTTCCAATGATGAGCACTTTTAAAGTTCTGCTATGTGGCGCGGTATTATCCCGTATTG
ACGCCGGGCAAGAGCAACTCGGTCGCCGCATACACTATTCTCAGAATGACTTGGTTGAGTA
CTCACCAGTCACAGAAAAGCATCTTACGGATGGCATGACAGTAAGAGAATTATGCAGTGCT
GCCATAACCATGAGTGATAACACTGCGGCCAACTTACTTCTGACAACGATCGGAGGACCGA
AGGAGCTAACCGCTTTTTTGCACAACATGGGGGATCATGTAACTCGCCTTGATCGTTGGGA
ACCGGAGCTGAATGAAGCCATACCAAACGACGAGCGTGACACCACGATGCCTGTAGCAAT
GGCAACAACGTTGCGCAAACTATTAACTGGCGAACTACTTACTCTAGCTTCCCGGCAACAA
TTAATAGACTGGATGGAGGCGGATAAAGTTGCAGGACCACTTCTGCGCTCGGCCCTTCCGG
CTGGCTGGTTTATTGCTGATAAATCTGGAGCCGGTGAGCGTGGGTCTCGCGGTATCATTGCA
GCACTGGGGCCAGATGGTAAGCCCTCCCGTATCGTAGTTATCTACACGACGGGGAGTCAGG
CAACTATGGATGAACGAAATAGACAGATCGCTGAGATAGGTGCCTCACTGATTAAGCATTG
GTAACTGTCAGACCAAGTTTACTCATATATACTTTAGATTGATTTAAAACTTCATTTTTAATT
TAAAAGGATCTAGGTGAAGATCCTTTTTGATAATCTCATGACCAAAATCCCTTAACGTGAG
TTTTCGTTCCACTGAGCGTCAGACCCCGTAGAAAAGATCAAAGGATCTTCTTGAGATCCTTT
TTTTCTGCGCGTAATCTGCTGCTTGCAAACAAAAAAACCACCGCTACCAGCGGTGGTTTGTT
TGCCGGATCAAGAGCTACCAACTCTTTTTCCGAAGGTAACTGGCTTCAGCAGAGCGCAGAT
ACCAAATACTGTTCTTCTAGTGTAGCCGTAGTTAGGCCACCACTTCAAGAACTCTGTAGCAC
CGCCTACATACCTCGCTCTGCTAATCCTGTTACCAGTGGCTGCTGCCAGTGGCGATAAGTCG
TGTCTTACCGGGTTGGACTCAAGACGATAGTTACCGGATAAGGCGCAGCGGTCGGGCTGAA
CGGGGGGTTCGTGCACACAGCCCAGCTTGGAGCGAACGACCTACACCGAACT

### 2. Methods:

### 2.1 Carboxylated chitosan sedimentation treatment of small-volume E. coli fermentation broth

*E. coli* Top 10 strains containing the pUC19 plasmid was cultured overnight in LB medium to obtain the *E. coli* fermentation broth, sampled, and measured its OD600. Six portions of 1 mL of the fermentation broth were used for the sedimentation assay of carboxylated chitosan. Different amounts of carboxylated chitosan solution were added into 1 mL fermentation broth sequentially, and vortex mixed; then corresponding amounts of sodium alginate solution were added, , andsettled for 30 min after vortex mixing. Another 1 mL fermentation broth was used as blank control. The OD600s of the control group and the carboxylated chitosan treatment group were measured. The added amounts of carboxylated chitosan and sodium alginate are shown in Table 1. The sedimentation rate was calculated based on the OD600 before and after sedimentation: Sedimentation rate = (Original sample OD600 - sample OD600 after sedimentation)/ Original sample OD600.

### 2.2 Effect of carboxylated chitosan sedimentation treatment of small-volume samples on plasmid DNA extraction

Different treatment methods were used to treat the *E*. *coli* cells in the control group sample and **the samples of experimental groups 1, 2, and 3** prepared by the method in section 2.1. The specific processes are as follows: (1) Control group: the bacterial solution was centrifuged at 3500g for 1 min, and the fermentation supernatant was removed to obtain the bacteria; (2) Experimental groups: A pipette was used to aspirate the fermentation supernatant after sedimentation to obtain the bacteria sedimented at the bottom of the centrifuge tube(s). 250µL of bacteria resuspension solution and 1 µL of RNase A were used to resuspend the cells, and then 250 µL of lysis buffer was added, gently inverted to mix and lyse the cells. 350 µL neutralization solution was then added and gently inverted to mix. After centrifugation at 12000g, the supernatant was collected, and an appropriate amount of magnetic beads was added and mixed well so that the magnetic beads can adsorb the DNA in the supernatant. Then a magnet was used to adsorb the magnetic beads and remove the supernatant. 70% ethanol was then added to wash the magnetic beads twice, dried, and the magnetic beads were eluted with 50 µL of ultrapure water to obtain plasmid DNA. The concentration of plasmid DNA was measured by a spectrophotometer and the yield of plasmid DNA was calculated.

### 2.3 Comparison of the Sedimentation Effects of Carboxylated Chitosan and Chitosan in Large-Volume E. coli Fermentation Broths

In two 250 mL conical shake flasks, 150 mL LB medium was added respectively to overnight culture the *E. coli* Top 10 strains containing the pUC19 plasmid. The *E. coli* fermentation broth was obtained, sampled, and measured for OD600. A small test preferred amount of flocculant was added to the carboxylated chitosan sedimentation groups: 8% carboxylated chitosan and 8% sodium alginate; and the amount of flocculant preferred in the published patent (CN109439584A) was added to the chitosan sedimentation groups: 4% chitosan and 2% sodium alginate. The adding processes and operations of the two experimental groups are as follows:
Carboxylated chitosan group: 12 ml (adding amount: 8%) carboxylated chitosan solution was added into the shake flask and shaken horizontally by hand for 30 s, and then the same volume of sodium alginate solution (adding amount: 8%) was added and shaken manually for 30s. Placed it at a low temperature for 4 h to allow the sufficient sedimentation *of E. coli* cells. Then the supernatants were taken respectively to measure OD600s and calculate the sedimentation rates.

Chitosan groups: 6 ml (adding amount: 4%) chitosan solution was added into the shake flask and rapidly mixed on a shaker at a rate of 120-150rpm for 2-3 min, and then 3 ml of sodium alginate solution (adding amount: 2%) was added and slowly mixed on a shaker at a rate of 50-60rpm for 2-5 min. Placed at a low temperature for 4 h to allow the sufficient sedimentation *of E. coli* cells. Then, the supernatants were taken to measure OD600s and calculate the sedimentation rates.

The supernatant was slowly poured out from the shake flask manually, the volume of the remaining bacterial solution was measured with a measuring cylinder, and the culture supernatant removal rate was calculated as follows: Sedimentation rate=(Original sample OD600 - sample OD600 after sedimentation ) / original sample OD600; culture supernatant removal rate = (Culture volume - volume of bacterial solution after sedimentation )/ culture volume.

### 2.4 Effects of carboxylated chitosan and chitosan treatment large-volume samples on plasmid DNA extraction

The concentrated bacterial solutions obtained after carboxylated chitosan and chitosan sedimentation were used for plasmid DNA extraction assay. 150 mL bacteria obtained by overnight culture *of E. coli* bacterial solution and centrifuged at 3500g for 10min were used as the control group. Since the volume of the remaining bacterial solution after the chitosan treatment was large, an electric pipette was used to carefully remove the culture medium that was not poured out completely, so that the volume of the final concentrated bacterial solution was the same as that of the carboxylated chitosan treatment group (about 10 ml) . 10 ml of bacteria resuspension solution was added to the bacteria of control group obtained by centrifugation to fully resuspend the bacteria without agglomeration. Subsequently, 10 µL RNase A was added to the bacterial solutions of the control group and the experimental group, shaken horizontally for 10s; then 10 mL lysis buffer was added, shaken horizontally for 30s to lyse the cells; 12 µL neutralization solution was then added, shaken horizontally for 30s. The neutralization solution in the shake flask was transferred to a 50 ml centrifuge tube, and centrifuged at 3500g for 10 min. The supernatant was then collected, and an appropriate amount of magnetic beads was added and mixed to allow the magnetic beads to adsorb the DNA in the supernatant. Then a magnet was used to adsorb the magnetic beads and remove the supernatant. 70% ethanol was then added to wash the magnetic beads twice, and after the ethanol was fully evaporated, 1000 µL of ultrapure water was used to elute the magnetic beads to obtain plasmid DNA. The concentration of plasmid DNA was detected by spectrophotometer and the yield of plasmid DNA was calculated. Plasmid bands and supercoil were observed by agarose gel electrophoresis. The experimental procedures of agarose gel electrophoresis were based on the Molecular Cloning: A Laboratory Manual (Fourth Edition), Michael R. Green, Joseph Sambrook, Cold Spring Harbor Laboratory Press, 2012*).*

### 2.5 Effects of the carboxylated chitosan alone treatment of large-volume samples on bacterial sedimentation and plasmid DNA extraction

In two 250 mL conical shake flasks, 150 mL of LB medium was added respectively to overnight culture the *E. coli* Top 10 strains containing the pUC19 plasmid. The *E*. *coli* fermentation broth was obtained, sampled, and taken to measure OD600. A small test preferred amount of flocculant was added to the experimental groups: 8% carboxylated chitosan; and an equal volume of ultrapure water was added to the control groups. The adding processes and operations of the two experimental groups are as follows:
Experimental group: 12 ml (adding amount: 8%) carboxylated chitosan solution was added into the shake flask and shaken horizontally by hand for 30s, placed at a low temperature to allow the sufficient sedimentation *of E. coli* cells. At 4h and 6h, the supernatants were taken to measure OD600s and calculate the sedimentation rates.

Control group: 12 ml ultrapure water was added to the shake flask, shaken horizontally by hand for 30s, and placed at a low temperature. The supernatants were taken at 4h and 6h to measure OD600s and calculate the sedimentation rates.

The supernatant was slowly poured out from the shake flask manually, the volume of the remaining bacterial solution was measured with a measuring cylinder, and the culture supernatant removal rate is calculated as follows: Sedimentation rate= (Original sample OD600 - sample OD600 after sedimentation ) / original sample OD600 ; culture supernatant removal rate = (Culture volume - volume of bacterial solution after sedimentation ) / culture volume

### 3. Experimental results and analysis

### 3.1 Sedimentation effect of carboxylated chitosan on small-volume E. coli fermentation broth

As described in Method section 2.1, different amounts of carboxylated chitosan and sodium alginate were used to study the effect on the bacterial sedimentation effect of the overnight-cultured 1 mL *E. coli* bacterial solution, and the results are shown in Table 1. Within a certain range, with the increase of the amounts of added carboxylated chitosan and sodium alginate, the bacterial sedimentation rate showed an upward trend. When the amounts of added carboxylated chitosan and sodium alginate were 8% and 8% respectively, the best bacterial sedimentation effect could be achieved, reaching 93.5%. When the amounts of the two added flocculants continued to increase, the sedimentation effect did not further improve, but showed a downward trend. Therefore, in subsequent studies, 8% carboxylated chitosan and 8% sodium alginate were adopted as sedimentation conditions.

**Table 1 Effects of different adding amounts of carboxylated chitosan and sodium alginate on bacterial sedimentation in 1 mL of E. coli fermentation broth**

| No. | Carboxylated chitosan (%) | Sodium alginate(%) | OD600 (OriginalOD600 (afterSedimentation sample) sedimentation) rate (%) | | |
|---|---|---|---|---|---|
| Control group | 0 | 0 | 3.39 | 3.39 | 0.00 |
| Experimental Group 1 | 4 | 2 | 3.39 | 1.24 | 63.42 |
| Experimental Group 2 | 8 | 4 | 3.39 | 0.94 | 72.27 |
| Experimental Group 3 | 8 | 8 | 3.39 | 0.22 | 93.51 |
| Experimental Group 4 | 16 | 8 | 3.39 | 1.94 | 42.77 |
| Experimental Group 5 | 16 | 16 | 3.39 | 2.43 | 28.32 |
| Experimental Group6 | 32 | 16 | 3.39 | 1.97 | 41.89 |

### 3.2 Effects of carboxylated chitosan and sodium alginate sedimented bacteria on plasmid extraction from small-volume samples

The sedimented bacteria obtained as above by carboxylated chitosan and sodium alginate experimental groups 1, 2, and 3 were selected for plasmid small-scale preparation test, and the bacteria collected by centrifugation were used as control (as shown in Section 2.2 of the Methods). The results are shown in Table 2. The control group demonstrated a plasmid yield of 9.4µg. The highest plasmid yield of 14.5 µg was achieved in experimental group 3, wherein a 8% carboxylated chitosan and sodium alginate were used to sediment the bacteria, which is 1.5 times of that of the control group. The plasmid yield increased linearly with the increase of bacterial sedimentation rate. The plasmid DNA A260/A280 obtained from the small-scale extraction of the control group and the experimental groups were both between 1.8 and 2.0, and without RNA and protein contamination, which can be used for the next step of research. It is worthly noticed that the plasmid yield obtained by sedimentation collection of bacteria under small-volume conditions is significantly higher than that of centrifugation collection. Carboxylated chitosan or sodium alginate may have a certain promoting effect on the plasmid yield.

**Table 2 Effects of carboxylated chitosan and sodium alginate sedimentation on small-scale plasmid extraction**

| No. | Carboxylated chitosan (%) | Sodium alginate (%) | DNA yield (µg) | A260/A280 |
|---|---|---|---|---|
| Control group | 0 | 0 | 9.4 | 1.90 |
| Experimental Group 1 | 4 | 2 | 9.7 | 1.91 |
| Experimental Group 2 | 8 | 4 | 12.2 | 1.91 |
| Experimental Group 3 | 8 | 8 | 14.5 | 1.89 |

### 3.3 Sedimentation Effects of Carboxylated Chitosan and Chitosan on Large-Volume E. coli Fermentation Broth

The optimal conditions for sedimentation of bacteria with carboxylated chitosan obtained from the small-scale extraction in Method 2.1 of this study: 8% carboxylated chitosan and sodium alginate, and the optimal conditions for sedimentation of bacteria with chitosan and sodium alginate reported in the published patent application (CN109439584A): 4% chitosan and 2% sodium alginate, were used in a sediment assay for a 150 mL of overnight cultured *E. coli* bacterial solution, and the results are shown in Table 3. As can be seen from Table 3, where the OD600s of the original fermentation broth were similar, the sedimentation rate of chitosan on bacteria is slightly higher than that of carboxylated chitosan, which are 98.4% and 92.7% respectively. However, when the culture supernatant is removed by direct pouring, the culture medium removal rate of the carboxylated chitosan group is significantly higher than that of the chitosan group, which are 93% and 62% respectively. After the culture supernatant is removed by direct pouring, the volume of the remaining bacterial solution of the carboxylated chitosan treatment group is only 10.5 mL. The high culture medium removal rate after sedimentation allows the samples sedimented with carboxylated chitosan to be processed automatically in batches by means of an automated robotic arm, which is advantageous for the system integration of the entire downstream automated operation after bacterial sedimentation.

**Table 3 Sedimentation results of carboxylated chitosan and chitosan on large-volume E. coli**

| No | Bacteria collection method | Fermentation broth volume (mL) | Fermentation broth OD600 | OD600 of Fermentation supernatant after sedimentation | Sedimentation rate | Volume of bacterial solution after sedimentation (ml) | Culture medium removal rate |
|---|---|---|---|---|---|---|---|
| Experimental Group 1 | Chitosan sedimentation | 150 | 3.65 | 0.06 | 98.4% | 57.5 | 62% |
| Experimental Group 2 | Carboxylated Chitosan sedimentation | 150 | 3.61 | 0.27 | 92.7% | 10.5 | 93% |

### 3.4 Effects of carboxylated chitosan and chitosan treatment of large-volume samples on plasmid DNA extraction

The yield of plasmid DNA prepared by alkaline lysis of the *E. coli* bacterial solution obtained after sedimentation in 3.3 was investigated. The plasmid DNA prepared by centrifugation was used as a control. Since the volume of the bacterial solution of chitosan group was large after sedimentation, the remaining culture medium was manually removed using a pipette to reduce the final volume of bacterial solution to about 10mL. Then 10 µL RNase A was added into to the bacterial solution, shake horizontally to mix evenly, then alkaline lysis solution was added to lyse the bacteria. Plasmid DNA was then extracted according to the method described in 2.4. The yield of extracted plasmid DNA is shown in Table 4.

**Table 4 Plasmid yield of carboxylated chitosan and chitosan sedimentation of E. coli**

| No. | Bacteria collection method | Plasmid DNA yield (µg) |
|---|---|---|
| Control group | Collecting bacteria by centrifugation | 324.5 |
| Experimental Group 1 | Chitosan sedimentation | 37.2 |
| Experimental Group 2 | Carboxylated chitosan sedimentation | 330.5 |

The extraction results in Table 4 indicates that the plasmid yields obtained from plasmid extraction to bacteria sedimented by carboxylated chitosan is comparable to plasmid extraction to bacteria collected by centrifugation, which are 330.5 µ g and 324.5 µg respectively. Chitosan sedimentation significantly reduced the efficiency of plasmid extraction from the bacteria, with a plasmid yield of only 37.2 µg, which is about 11% of that of the carboxylated chitosan treatment group. Visual observation of the lysed bacteria obtained by sedimentation revealed that chitosan and sodium alginate cause *E*. *coli* to form larger flocculent bacterial mass, which were difficult to disperse evenly, and further resulted in insufficient lysis during alkaline lysis. The *E. coli* obtained by carboxylated chitosan and sodium alginate sedimentation had a fine appearance and could be quickly and evenly after dispersed by manual shaking, without affecting the subsequent alkaline lysis (results not shown). Therefore, the use of chitosan and sodium alginate to sediment bacteria is more suitable for removing bacteria from fermentation broth and collecting fermentation supernatant; while the sedimentation of bacteria by carboxylated chitosan and sodium alginate not only can be used to collect fermentation supernatant, but also can be used for subsequent bacteria treatment, such as extracting biological products inside the bacteria: plasmid DNA, RNA, intracellular proteins, *etc.*

According to the A₂₆₀ /A₂₈₀ test results of plasmid DNA (Table 2) and the plasmid supercoil ratio results shown in the agarose gel electrophoresis diagram (Figure 1), it can be seen that the method of collecting bacteria by carboxylated chitosan has no impact on the quality of the extracted plasmid DNA.

### 3.5 Effects carboxylated chitosan alone treatment of large-volume samples on bacterial sedimentation and plasmid DNA extraction

Effects of using carboxylated chitosan alone on the *E. coli* bacterial sedimentation and plasmid DNA extraction yield were investigated. The samples without carboxylated chitosan were used as sedimentation efficiency control and plasmid yield control. The results are shown in Table 5.

**Table 5 Effects of carboxylated chitosan alone on the large-volume bacterial solution sedimentation and t plasmid DNA extraction yield**

| No. | Bacteria collection method | Bacterial sedimentation rate after placing 4h | Bacterial sedimentation rate after placing 6h | Plasmid DNA yield (µg) |
|---|---|---|---|---|
| Control group | Centrifugation | 0% | 0% | 320.5 |
| Experimental group | Carboxylated chitosan | 80.5% | 95.2% | 328.1 |

As can be seen from Table 5, after 4 hours of sedimentation, the bacterial sedimentation rate of the experimental group reached 80.5%, which is lower than the bacterial sedimentation rate of 92.7% achieved after 4 hours of carboxylated chitosan and sodium alginate sedimentation (Table 3) . However, when using 8% carboxylated chitosan alone after 6 hours of sedimentation, a comparable bacterial sedimentation effect (95.2%) to that of the combination of carboxylated chitosan and sodium alginate at 4 hours can be achieved. Although the sedimentation time of using carboxylated chitosan alone is extended by 2 hours compared to the combined use of two flocculants, it simplifies the reagent adding processes and avoids the risk of inaccurate liquid addition and pipeline blockage caused by the high viscosity of sodium alginate during the automated operation, thereby offering greater application values.

In addition, when the volume or batch size of the *E. coli* bacterial solution to be processed needs to be scaled up, both the centrifugation method and the membrane filtration method face challenges such as limited batch processing capacity, the need for extensive manual operations, difficultly in achieving automation, and increased costs due to the requirement of adding corresponding centrifugation or membrane filtration equipment. The sedimentation method of the present disclosure only requires the addition of 1-2 flocculants, and the separation of the bacteria and the fermentation medium can be achieved by gravity sedimentation under low temperature conditions. The method is simple, low cost, and easily scalable for automated batch processing, which is superior to the centrifugation and membrane filtration methods.

### 4. Summary of results:

As mentioned above, the inventor unexpectedly found that the bacteria sedimentation in the *E*. *coli* fermentation broth can be effectively achieved by using water-soluble carboxylated chitosan and sodium alginate or using carboxylated chitosan alone, avoiding centrifugation or membrane filtration, and easy to operate. After the *E*. *coli* fermentation broth is treated with the flocculant(s), the fermentation supernatant can be removed by a pouring to obtain the sedimented cell. This process can be automated by a mechanical arm. The bacteria precipitate obtained by the sedimentation method of the present disclosure is relatively dispersed, does not agglomerate to form floccules, and is conducive to cell lysis and subsequent extraction of intracellular plasmid DNA , RNA and protein.

It is obvious to those skilled in the art that various modifications and changes can be made to the various embodiments described herein without departing from the spirit or scope of the teachings herein. Therefore, it is intended that various embodiments cover other modifications and changes of various embodiments within the scope of the teachings herein.

### Example 2: Pichia pastoris sedimentation

### 1. Reagents

YPD medium: 1% yeast extract (Thermo Scientific, LP0021B), 2% peptone (Thermo Scientific , LP0042B ), 2% dextran (Sinopharm Reagent, 63005518 ).

Carboxylated chitosan solution: 1.51 g carboxylated chitosan (Aladdin Scientific, C105800 ) was dissolved in 100 mL ultrapure water to obtain a 1.5% carboxylated chitosan aqueous solution.

### 2. Method

*Pichia pastoris* GS115 strains were cultured in 4 mL YPD medium to obtain *Pichia pastoris* GS115 fermentation broth, and samples were taken to measure OD600s. Different amounts of carboxylated chitosan solution were added to the fermentation broth, vortex mixed, and then placed at 4°C. Fermentation broth without carboxylated chitosan was taken as a blank control. The OD600s of the control group and the carboxylated chitosan treatment groups were measured hourly. The sedimentation rate was calculated based on the OD600 before and after sedimentation. Sedimentation rate = (Original sample OD600 - sample OD600 after sedimentation) / Original sample OD600.

### 3. Result

As described in the method section, different amounts of carboxylated chitosan were added to affect the bacterial sedimentation of 4 mL *Pichia pastoris* bacterial solution. The results are shown in Table 6.

**Table 6 Effects of different amounts of carboxylated chitosan on bacterial sedimentation in Pichia pastoris fermentation broth**

| No. | Carboxylated chitosan ( %) | Sedimentation rate after placing 1h (%) | Sedimentation rate after placing 2h (%) |
|---|---|---|---|
| Control group | 0 | 9.1% | 60.5% |
| Experimental Group 1 | 4 | 32.3% | 63.7% |
| Experimental Group 2 | 8 | 50.5% | 94.2% |
| Experimental Group 3 | 16 | 52.9% | 92.5% |

Within a certain range, with the increase of carboxylated chitosan addition, the bacterial sedimentation rate showed an upward trend. When the carboxylated chitosan addition was 8%, the best bacterial sedimentation effect could be achieved, reaching 94.2%. Further increasing the amount of carboxylated chitosan did not further improve the sedimentation effect.

This example shows that carboxylated chitosan has a favorable effect on the cell sedimentation of *Pichia pastoris* cells, which can be used for collecting *Pichia pastoris* cells or collecting culture medium supernatant after sedimentation for subsequent protein purification and other applications.

## Claims

**1.** A method for collecting bacteria from bacterial or fungal fermentation culture broth, **characterized in that** the method comprises the steps of:
a) mixing the fermentation culture broth obtained from bacterial or fungal fermentation culture with a carboxylated chitosan to obtain a mixture;
b) adding sodium alginate solution into the mixture and mixing homogeneously; and
c) precipitating to obtain bacterial precipitate or fermentation culture supernatant.

**2.** The method according to claim 1, wherein the step b) is omitted.

**3.** The method according to claim 1 or 2, further comprising the step d) of recovering the bacteria or the fermentation culture supernatant after step c).

**4.** The method according to claim 1 or 2, wherein the bacteria are selected from *Bacillus, Escherichia coli, Lactobacillus, Lactococcus,* preferably *Escherichia coli* or *Bacillus subtilis*; the fungi are selected from yeast, preferably baker's yeast, feed yeast, *Saccharomyces cerevisiae, Pichia pastoris*; *Candida*; mucedine, more preferably *Rhizopus, Aspergillus,* most preferably *Saccharomyces cerevisiae* and *Pichia pastoris.*

**5.** The method according to claim 1 or 2, wherein the amount of carboxylated chitosan added is 1%-32% by weight, preferably 1-16% by weight, more preferably 4%-8% by weight, and most preferably 8% by weight of the fermentation culture broth. 5.

**5.** The method according to claim 1, wherein the amount of sodium alginate mixed in step b) is 0%-16% by weight, preferably 0 %-8% by weight, and more preferably 0% or 8% by weight of the fermentation culture broth.

**6.** The method according to claim 1, wherein the carboxylated chitosan and sodium alginate added to the fermentation culture broth are 8% by weight of the fermentation culture broth, respectively, and preferably the fermentation culture broth is selected from *Escherichia coli* and *Pichia pastoris* fermentation culture broth.

**7.** The method according to claim 2, wherein the carboxylated chitosan added to the fermentation culture broth is 8% by weight of the fermentation culture broth, and preferably the fermentation culture broth is *Escherichia coli* and *Pichia pastoris* fermentation culture broth.

**8.** The method according to claim 1 or 2, wherein the carboxylated chitosan is selected from chitosan in which amino (-NH₂) , or hydroxyl (-OH), or amino (-NH₂) and hydroxyl (-OH) are modified by carboxylation groups, wherein the carboxylation groups are C1-C6 linear or branched alkyl groups substituted with 1-3 carboxyl groups, preferably 3 carboxyl groups, more preferably 2 carboxyl groups, and most preferably 1 carboxyl group; the linear or branched alkyl groups preferably have 1-6 carbon atoms.

**9.** The method of claim 4, wherein the strains of *E. coli* comprise *E*. *coli* TOPIC, *E*. *coli* JM109, *E*. *coli* HB101, *E*. *coli* DH5α, *E*. *coli* BL21 (DE3), and other derivative strains of *E*. *coli* K strains and B strains, including *E. coli* DH10B, *E. coli* JM110, *E. coli* MC1061, *E. coli* MG1655, *E. coli* Stbl2, *E. coli* Stbl3, *E. coli* T-Fast, *E. coli* XL1 Blue, *E. coli* Rosetta 2 (DE3), etc., or wherein the strains of yeast comprise *Saccharomyces* cerevisiae and Pichia *pastoris.*

**10.** The method according to claim 3, wherein the recovery in the step d) is selected from pouring the culture medium or pouring the culture medium by an automated robot arm to obtain bacteria or culture supernatant, adsorbing DNA/ RNA or protein by magnetic beads, and washing DNA/RNA or protein, preferably pouring, more preferably manually pouring the culture supernatant or pouring the culture supernatant by an automated robot arm.
